# EUROPEAN PATENT SPECIFICATION

(11) **EP 4 404 774 B1**
(45) Date of publication and mention of the grant of the patent: **12.02.2025**
(21) Application number: 22778030.1
(22) Date of filing: 20.09.2022
(51) Int. Cl.: A24B 13/00, A24B 15/30

(54) **ACTIVE INGREDIENT DELIVERY SYSTEM**
WIRKSTOFFFREISETZUNGSSYSTEM
SYSTÈME DE DISTRIBUTION DE PRINCIPE ACTIF

(30) Priority: 20.09.2021 US 202163246089 P
(43) Date of publication of application: 31.07.2024
(60) Divisional application: 25150465.0
(73) Proprietor: Opes Corporation OY, 02200 Espoo (FI)
(72) Inventor: JÄRVENPÄÄ, Janne, 02360 Espoo (FI)
(74) Representative: Boco IP Oy Ab
(86) International application number: PCT/FI2022/050629
(87) International publication number: WO 2023/041848

(56) References cited:
- WO-A1-2005/115180
- RU-C1- 2 745 039
- US-A1- 2016 101 108
- US-A1- 2019 059 438
- US-B2- 10 821 147
- US-B2- 9 027 567

## Description

### BACKGROUND

The subject matter disclosed herein relates generally to an active ingredient delivery system for delivering an active ingredient to a user. More particularly, the subject matter disclosed herein relates to a paper-based product applied, for example, by screen printing, with a matrix having an active ingredient component, such as nicotine. The matrix may further include a softening agent, such as glycerin. The active ingredient may absorb or be released into a user when the matrix-containing, paper-based substrate is placed on a user's gums. The active ingredient delivery system may further include a flavor component for imparting a flavor to the active ingredient delivery system.

Existing active ingredient delivery systems typically have harmful side effects or undesirable qualities for consumers. For example, nicotine is often delivered to a user via smoking cigarettes, which have many well-known harmful side effects. Alternatively, nicotine may be delivered via chewing gum substances or, alternatively, via portioned sachets containing tobacco and known as snus, however, chewing gum and snus are often not discrete nor appropriate in all settings. Variations of snus, so-called "white pouch products," wherein the tobacco contents of the snus sachet are replaced with nicotine and flavor absorbed into cellulose pulp, have similar disadvantages in that they are not discrete or appropriate in all settings. Such white pouch products do have fewer harmful, attending side effects, but are known by those having skill in the art to have a maximum moisture content of approximately 30%, which limits the shelf-life and prevents optimal absorption of active ingredient by a user. Thus, there is a need for a discrete active ingredient delivery system that is not harmful to the user and has optimal user properties.

As another example, caffeine is often ingested via drinkable liquids (e.g., coffee, energy drinks) or in pill form. Drinkable liquids containing caffeine are also not discrete nor appropriate in all settings and are often packaged in plastic or coated aluminum containers that can create a great deal of environmental waste relative to paper-based products. Further, users are required to carry around relatively bulky liquid containers. Caffeine in pill form is similarly inconvenient because of the need of a drinkable liquid to assist in ingesting.

Patent document US10821147B2 discloses a printable ink composition comprising a purified cannabinoid, a purified terpene, an excipient and a solvent, the printable ink composition being printed on a substrate such as paper, sugar sheet and a dissolvable film.

Patent document RU2745039C1 discloses an edible biodegradable product for oral consumption of nicotine, which is a polymer substrate coated with a solution of nicotine, its pharmaceutically acceptable salt or its analogue, characterized in that the product is an edible paper in the form of a strip soaked in a solution of nicotine, its pharmaceutically acceptable salt or its analogue.

Patent document US2016101108 discloses a printed oral dosage form of vitamin(s) and/or dietary mineral(s), comprising at least two of vitamin(s) and/or mineral(s), wherein at least one vitamin and/or dietary mineral is included in the substrate material and at least one vitamin and/or dietary mineral is printed on the substrate.

The active ingredient delivery system of the present invention provides a convenient, discrete, paper-based product that delivers an active ingredient to a user by applying the paper-based product to the gums. Preferably, the delivery system of the present invention creates little to no harmful side effects to the user and includes optimal shelf-life and user properties.

### SUMMARY

It is to be understood that this summary is not an extensive overview of the disclosure. This summary is exemplary and not restrictive, and it is not intended to identify key or critical elements of the disclosure or to delineate the scope thereof. The sole purpose of this summary is to explain and exemplify certain concepts of the disclosure as an introduction to the following detailed description.

An active ingredient delivery system is disclosed. The system includes a paper-based substrate comprising fibers that are not dissolvable but are biodegradable and a matrix. The matrix includes an active ingredient component and a carrier. The matrix is disposed by printing onto the paper-based substrate. The matrix may optionally further include one or more of a flavor component, a softening agent, a viscosity regulator, an additional carrier, and a sweetener. The paper-based substrate has a length, a width, and a depth dimension such that the paper-based substrate, having the matrix disposed thereon, fits on a user's gums.

In another embodiment of the present invention, the active ingredient delivery system includes a paper-based substrate having multiple connected segments, such as strips. In one embodiment, the multiple segments are connected via perforations. The active ingredient delivery system further includes a matrix. The matrix may include an active ingredient component and a carrier. Further, the matrix may include a flavor component, a softening agent, and/or a sweetener. The carrier, the softening agent, the flavor component, and the sweetener are preferably food-grade. The matrix may be disposed on the paper-based substrate. In embodiments incorporating perforations, the perforations may allow a user to easily tear off a segment of the multiple segments of the paper-based substrate having matrix disposed thereon. Further, the segments each have a length, a width, and a depth dimension such that the segments when separated fit on a user's gums.

The present invention also includes a method for making an active ingredient delivery system. The method comprises the steps of providing a paper-based substrate comprising fibers that are not dissolvable but are biodegradable, printing a matrix onto the paper-based substrate, the matrix comprising an active ingredient component and a carrier and sizing the paper-based substrate having the matrix printed thereon to have a length, a width, and a depth dimension so that the paper-based substrate fits on a user's gums. In one aspect of such embodiments, reloading of matrix onto the substrate is accomplished between each printing run using dosing pumps. The method may further include the step of sizing the paper-based substrate having the matrix disposed thereon to have length, width, and depth dimensions so that the paper-based substrate fits on a user's gums.

In an alternative embodiment, the matrix may further include at least one of a flavor component, a softening agent, a viscosity regulator, an additional carrier, and a sweetener. In preferred embodiments of the disclosed method, the step of disposing a matrix onto the paper-based substrate may be accomplished via screen printing. In one aspect of such embodiments, reloading of the matrix onto the substrate may be accomplished between each screen printing run using dosing pumps. The method may further include the step of perforating the paper based substrate so that segments, such as strips, are joined and separated via the perforation. The segments may have a length, a width, and a depth dimension so that each segment fits on a user's gums for absorption or release of the active ingredient component into a user.

These and other features and their advantages will be apparent from a careful review of the detailed description below, accompanied by the following drawing.

### BRIEF DESCRIPTION OF THE DRAWING

The aspects described above, as well as other apparent aspects, advantages, and objectives of the present invention are apparent from the detailed description below in combination with the drawing, in which:
Fig. 1 is an image of an embodiment of the active ingredient delivery system with multiple connected segments in accordance with the present invention.
Fig. 2 is an image of an embodiment of the active ingredient delivery system in accordance with the present invention.

### DETAILED DESCRIPTION OF REPRESENTATIVE EMBODIMENTS

The present invention may be understood more readily by reference to the following detailed description, examples, drawings, and claims, and their foregoing and following descriptions.

As used in the specification and the appended claims, the singular forms "a," "an," and "the" include plural referents unless the context clearly dictates otherwise.

Unless otherwise stated, the following terms used in the specification and claims have the meanings given below.

As used herein, the term "matrix" refers to a combination of substances. A matrix as used herein may include one or more active ingredient components, one or more fluid or solid carriers, such as propylene glycol, one or more flavor components, a softening agent, such as glycerin, one or more viscosity regulators, and one or more sweeteners. A matrix may be solid or liquid by itself, in preparation to be disposed onto a paper-based product, but in liquid form for printing. "Liquid" as contemplated by this definition includes fluids, further including but not limited to gels and pastes as may be used in printing. Where a matrix is printed on a paper-based substrate, "matrix" may refer to one or more liquids that are printed together in one mixture in one or more printing runs or printed in separate mixtures containing different matrix components in separate printing runs. Whether a matrix is printed in one mixture or separate mixtures, when the present disclosure refers to component amounts of "a" or "the" matrix, it is intended that such measures be interpreted as a portion of total matrix (i.e. across the sum of all mixtures in a matrix), unless context dictates otherwise. A matrix may remain fluid or may form a dried, solid film or coating once disposed onto a paper-based product. A matrix may further partially or completely impregnate a paper-based product as or after it is applied onto a paper-based product.

As used herein, the term "paper-based product" refers to any cellulose- or cellulose pulp-based product that can maintain its structure when disposed with a matrix as defined herein. The "paper-based product" may be produced on a traditional or modified paper mill. In general, standard paper-based products used in the food and food packaging industry are contemplated with the use of the term "paper-based product," though the disclosure identifies preferred embodiments having specific component materials content that are particularly useful to the disclosed invention.

The term "moisture content" as it is used in relation to an active ingredient delivery system in the present disclosure refers to the percentage of the active ingredient delivery system by weight that comes from a liquid source. Moisture content is determined by first weighing a dry substrate, then disposing, such as by applying, materials, including a matrix, onto the substrate. The matrix-applied substrate is weighed again, promptly after application, and moisture content is determined by subtracting the dry substrate weight from the matrix-applied substrate weight, dividing that mount by the matrix-applied substrate weight, and multiplying by 100 to yield a percentage value. For example, a dry sheet is weighed and has a weight of 100 grams. After printing of a matrix onto the dried substrate, the matrix-applied substrate is weighed and has a weight of 181 grams. 181 grams matrix-printed sheet minus 100 grams dry substrate yields a weight of 81 grams liquid. 81 grams liquid divided by 181 grams (matrix-printed substrate weight) and multiplied by 100 yields a moisture content of about 45%. Any suitable, calibrated scale can be used to measure weight.

As used herein, the term "active ingredient component" refers to a substance that produces a chemical or biological effect in a user when absorbed or released into the body and is capable of being disposed on a paper-based product as defined herein. These materials include but are not limited to substances such as nicotine, CBD, THC, caffeine, ibuprofen, pharmaceuticals, vitamins, glucose or other natural or synthetic sugars, other substances, and mixtures thereof in pure form or contained in a liquid, solid, or other carrier. As used herein, composition amounts of "active ingredient component" refer to the amount of active ingredient component absent a solvent, vehicle, or other carrier, unless context dictates otherwise. "Active ingredient" is generally synonymous with "active ingredient component" as used herein.

As used herein, the term "food-grade" refers to any substance or component that is safe for human consumption/absorption.

As used herein, the term "CBD" refers to cannabidiol, a phytocannabinoid discovered in 1940. It is one of 113 identified cannabinoids in cannabis plants, along with tetrahydrocannabinol (THC). CBD accounts for approximately up to 40% of cannabis plant extract. The term CBD, as used herein, refers to CBD extract, which may contain impurities and other compounds inherent to the extract process.

As used herein, the term "THC" refers to tetrahydrocannabinol, a lipid found in cannabis plants. THC is the principal psychoactive constituent of cannabis plants and one of at least 113 total cannabinoids identified in the plant. Although the chemical formula for THC, C₂₁H₃₀O₂, describes multiple isomers, the term THC as used herein generally refers to the Delta-9-THC isomer, with chemical name (-)-trans-Δ⁹-tetrahydrocannabinol. It is contemplated, however, that the term THC as used herein, refers to THC extract, which may contain impurities, other isomers of the THC formula C₂₁H₃₀O₂, and other compounds inherent to the extract process.

As used herein, the term "softening agent" refers to a material that softens a substrate to preferably aid in the user's experience, for example, by improving or maintaining mouthfeel of a matrix-disposed paper-based substrate. The "softening agent" may also aid in ease of handling in the manufacturing process. Preferably the "softening agent" of the present invention is food-grade as defined herein. One exemplary softening agent contemplated in this disclosure is glycerin. Suitable softening agents may not significantly adversely affect the absorbency of paper-based substrates to which they are applied.

When used with respect to a component which may be found in a mixture, the term "substantially free of" means containing less than about 2 wt. % of the component based on the mixture weight.

The recitation of a numerical range using endpoints includes all numbers subsumed within that range (e.g., 1 to 5 includes 1, 1.5, 2, 2.75, 3, 3.80, 4, 5, etc.).

The terms "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

The accompanying figures illustrate certain of the foregoing aspects of the active ingredient delivery system, as well as additional aspects thereof. The figures represent embodiments of the invention and are not intended to limit the invention.

The subject matter disclosed includes an active ingredient delivery system. The system of the present invention preferably delivers an active ingredient, such as nicotine, CBD, THC, caffeine, ibuprofen, pharmaceuticals, sugars, vitamins, other substances, or mixtures thereof to a user by placing a matrix-disposed, paper-based product on the gums of the user so that the active ingredient is absorbed or released into the user's body.

Although primarily described herein in terms of delivering active ingredients to humans via the mouth and/or gums, it will be clear that the subject matter disclosed may have various other delivery locations and may also be used with non-humans, such as pets.

Reference now will be made in detail to embodiments and examples of the subject matter disclosed.

The present invention includes an active ingredient delivery system. The active ingredient delivery system comprises a paper-based substrate comprising fibers that are not dissolvable but are biodegradable and a matrix, wherein the matrix comprises an active ingredient component and a carrier,wherein the matrix is disposed on the paper-based substrate by printing the matrix onto the substrate and wherein the paper-based substrate has a length, a width, and a depth dimension such that the paper-based substrate, having the matrix disposed thereon, fits on a user's gums. The matrix may optionally further include one or more of a flavor component, a softening agent, a viscosity regulator, an additional carrier, and a sweetener.

In another embodiment of the present invention, the active ingredient delivery system includes a paper-based substrate comprising multiple connected segments and fibers that are not dissolvable but are biodegradable and a matrix, wherein the matrix comprises an active ingredient component and a carrier, wherein the matrix is printed on the paper-based substrate and wherein the multiple connected segments are connected to allow a user to easily separate a segment of the multiple connected segments of the paper-based substrate having matrix printed thereon and wherein each segment of the multiple connected segments has a length, a width, and a depth dimension such that each segment fits on a user's gums, optionally the multiple connected segments are connected via perforations. Further, the matrix may include a flavor component, a softening agent, and/or a sweetener. The carrier, the softening agent, the flavor component, and the sweetener are preferably food-grade. In embodiments incorporating perforations, the perforations may allow a user to easily tear off a segment of the multiple segments of the paper-based substrate having matrix disposed thereon.

The present invention also includes a method for making an active ingredient delivery system. The method includes the steps of providing a paper-based substrate comprising fibers that are not dissolvable but are biodegradable, printing a matrix onto the paper-based substrate, the matrix comprising an active ingredient component and a carrier and sizing the paper-based substrate having the matrix printed thereon to have a length, a width, and a depth dimension so that the paper-based substrate fits on a user's gums. In one aspect of such embodiments, reloading of matrix onto the substrate is accomplished between each printing run using dosing pumps.

In an alternative embodiment, the matrix may further include an active ingredient component and at least one carrier. Further, the matrix may include at least one of a flavor component, a softening agent, a viscosity regulator, an additional carrier, and a sweetener. In preferred embodiments of the disclosed method, the step of disposing a matrix onto the paper-based substrate may be accomplished via screen printing. In one aspect of such embodiments, reloading of the matrix onto the substrate may be accomplished between each screen printing run using dosing pumps. The method may further include the step of perforating the paper based substrate so that segments, such as strips, are joined and separated via the perforation. The segments have a length, a width, and a depth dimension so that each segment fits on a user's gums for absorption or release of the active ingredient component into a user.

### SUBSTRATE

In certain embodiments, the substrate is a paper-based product that can be formed off of a standard or modified paper mill. In certain embodiments, the substrate includes cellulose pulp. Cellulose pulp is an absorbent organic fiber that retains applied liquids well for the present application. In certain preferred embodiments, the substrate includes cellulose pulp and cotton fibers, for example, cotton linters. Cotton fibers are soft and can improve the comfort for a user inserting an active ingredient delivery system into their mouth.

In certain embodiments, the substrate may be made up of additional materials common to paper-based products in addition to or in lieu of the materials described above. Generally, absorbent materials are useful in the substrate described in the present disclosure. Fibers that are not dissolvable but are biodegradable are comprised in the paper-based substrate. For example, fibers having good absorbency, which are not dissolvable, but are biodegradable, like modal fiber, viscose, wool, lyocell, and ioncell may be used. Further, different types of cotton fibers or modified cellulose or cotton fibers may also be used. In general, the substrate must be made up of materials of sufficient structural and chemical integrity to bear a matrix disposed thereon.

In certain embodiments, the substrate can be sized such that it carries a safe, effective dose of the active ingredient component and also comfortably fits in a user's mouth, on a user's gums, between gum and cheek tissue, without showing significantly as a protrusion on the user's face, and without being visible to a third person. Accordingly, the thickness of the substrate will be such that an overall thickness of the active ingredient delivery system will be thin relative to white pouch products or snus. The active ingredient delivery system is further easy to store and transport. The thickness of the substrate is also important for its ability to carry the disposed matrix. If the substrate is too thin, then the substrate will not adequately support or contain the active ingredient component of the matrix. If the substrate is too thick or otherwise too large, it will not fit comfortably in a user's mouth, on a user's gums, between gum and cheek tissue. Further, a substrate that is otherwise too thick or too large or too thin will not adequately absorb, retain, and/or release to a user the liquid of a matrix that is disposed on the substrate. Generally, the paper forming the sachets of white pouch products is too thin to operate as a paper-based substrate as disclosed herein.

In embodiments, the dimensions of the substrate substantially determine the dimensions of the active ingredient delivery system. In preferred embodiments, the active ingredient delivery system substantially forms a rectangular prism with square or rounded edges and corners, having a length, a width, and a thickness (also referred to as a depth herein). In preferred embodiments, the length is at least 5 mm, more preferably at least 10 mm, more preferably at least 15 mm more preferably, at least 18 mm, more preferably at least 21 mm, more preferably at least 24 mm, and at most 75 mm, more preferably at most 60 mm, more preferably at most 50 mm, more preferably at most 35 mm, more preferably at most 32 mm, more preferably at most 29 mm, and more preferably at most 26 mm. In said embodiments, the width is at least 3 mm, more preferably at least 4 mm, more preferably at least 5 mm, more preferably at least 7 mm, more preferably at least 9 mm, and at most 20 mm, more preferably at most 17 mm, more preferably at most 15 mm, more preferably at most 13 mm, more preferably at most 11 mm, and more preferably at most 8 mm. In said embodiments, the thickness is at least 0.2 mm, more preferably at least 0.3 mm, more preferably at least 0.5 mm, more preferably at least 0.7 mm, more preferably at least 1.0 mm, more preferably at least 1.2 mm, and at most 3.0 mm, more preferably at most 2.7 mm, more preferably at most 2.3 mm, more preferably at most 2.1 mm, more preferably at most 2.0 mm, and more preferably at most 1.5 mm.

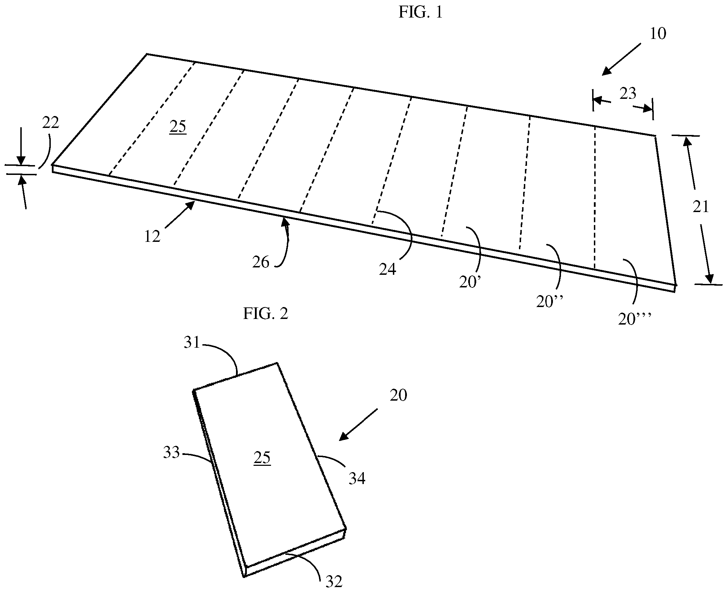

The substrate may further be perforated, as shown in FIG.1, to allow for a user to tear off an individual dose of the active ingredient delivery system.

### MATRIX

The matrix may include an active ingredient component and may further include a flavor component and/or additional components. In the active ingredient delivery system, the matrix may be disposed on the substrate as a single mixture, or the different constituent components of the matrix or mixtures thereof may each be disposed as separate mixtures, each simultaneously or at different times.

In embodiments, the active ingredient component is capable of absorbing or releasing into a user. The active ingredient component may include, for example, nicotine, CBD, THC, caffeine, ibuprofen, pharmaceuticals, vitamins, glucose or other natural or synthetic sugars, other substances, and mixtures thereof.

In certain embodiments, the matrix further includes at least one carrier. The carrier may be any solvent, fluid, or vehicle that is safe for human consumption/absorption. The carrier is selected such that the carrier is compatible with the active ingredient component and the active ingredient component is miscible or dispersible in the carrier. In certain embodiments, the carrier may be water, ethanol, propylene glycol, or the like, or mixtures thereof. In a preferred embodiment, the carrier includes propylene glycol.

In certain embodiments, the active ingredient component is present in the matrix in amounts sufficient to allow for an effective dose to be delivered such as via absorption through a user's gums from the active ingredient delivery system. A person having skill in the art will understand that changing the volume, purity, and concentration of the active ingredient component used in the active ingredient delivery system will affect the effectiveness and intensity of the dose delivered.

In some embodiments, the active ingredient component may be present in the matrix in an amount of 0.02 wt. % to 30 wt. % by weight of the matrix, in further embodiments from 0.2 wt. % to 15 wt. % by weight of the matrix, in further embodiments from 0.5 wt. % to 8 wt. % by weight of the matrix, in further embodiments from 0.5 wt. % to 3 wt. % by weight of the matrix, and in still further embodiments from 1 wt. % to 2 wt. % by weight of the matrix. These embodiments are particularly beneficial when nicotine is the active ingredient component.

In some embodiments, the matrix includes 0.05 to 20 wt.% flavor component as a percentage of the matrix weight, in further embodiments from 0.2 to 18 wt.%, in further embodiments from 0.5 to 10 wt. %, in further embodiments from 1 to 7 wt. %, in further embodiments from 1.5 to 5 wt. %, and in still further embodiments from 1.8 to 3 wt. % flavor component as a percentage of matrix weight.

In some embodiments, the matrix may include a softening agent, such as glycerin. In some embodiments, a carrier, flavor carrier, or additional carriers make up the balance of the matrix weight.

In certain preferred embodiments, the active ingredient component is nicotine and is present in the matrix in solution with a carrier, where the combined nicotine-carrier solution is present in the matrix in an amount of 1.5 wt. % to 30 wt. % by weight of the matrix. In further preferred embodiments, the nicotine-carrier solution makes up 4 wt. % to 20 wt. %, 5 wt. % to 15 wt. %, or 6 to 10 wt. % as a percentage of the weight of the matrix. In preferred embodiments, the carrier may be propylene glycol.

In certain embodiments, the active ingredient component may be liquid nicotine, nicotine oil, crystalized nicotine, a nicotine salt, or a mixture thereof.

In certain embodiments where the active ingredient component is nicotine, each portion of substrate intended to provide an individual dose includes about 1-30 mg of nicotine. For example, a portion of substrate may include 1 mg of nicotine, alternatively 4 mg of nicotine, alternatively 8 mg of nicotine, alternatively 10 mg of nicotine, or further alternatively 12 mg of nicotine. In certain preferred embodiments, the propylene glycol is used as a carrier for the active ingredient component.

In certain embodiments, the flavor component may be any food approved compound that carries a flavor. In certain embodiments, the flavor component is wintergreen, spearmint, cool mint, peppermint, citrus, cinnamon, coffee, licorice, lime, bergamot, menthol, vanilla, blueberry, raspberry, synthetic compounds used to simulate these flavors, or a mixture thereof. In preferred embodiments, the flavor component is incorporated into a flavor carrier fluid or flavor carrier, which can also form a part of the matrix. The flavor carrier fluid is selected such that the flavor component is miscible or dispersible in the flavor carrier fluid. Exemplary, suitable flavor carriers include water, propylene glycol, ethanol, mixtures thereof, or the like. The flavor carrier may be the same or different than any carrier compatible with the active ingredient component. In embodiments, the flavor component may be prepared in a mixture with the active ingredient component, or the flavor component may be part of its own mixture, prepared separately from the active ingredient component.

In certain embodiments, the matrix includes an active ingredient component, and optionally, one or more of a flavor component, a softening agent, a viscosity regulator, an additional carrier, and/or a sweetener.

The matrix can be provided with a softening agent, which preferably aids in a user's experience, for example, by improving or maintaining mouthfeel of a matrix-disposed substrate. A softening agent may improve mouthfeel by reducing or eliminating mucosal irritation with the active ingredient delivery system. A softening agent may also aid in processing of a substrate during application of a matrix to the substrate by making the substrate more pliable. In embodiments, the matrix comprises at most 80 wt. % softening agent, in more preferable embodiments, at most 60 wt. %, in more preferable embodiments, at most 50 wt. %, in more preferable embodiments, at most 45 wt. %, and in still more preferable embodiments, at most 40 wt. %. In embodiments, the matrix comprises at least 2 wt. % softening agent, in more preferable embodiments, at least 5 wt. % softening agent, in more preferable embodiments, at least 12 wt. % softening agent, in more preferable embodiments, at least 15 wt. % softening agent, and in still more preferable embodiments, at least 18 wt. % softening agent. One suitable softening agent is glycerin. In preferred embodiments, food-grade glycerin is used. In alternate embodiments, glycerin, diethylene glycol, propylene glycol, ceramides, naturally derived oils and butters such as shea butter, jojoba oil, cocoa butter or avocado oil, or mixtures thereof may also be used.

The matrix can be provided with a viscosity regulator, which can be used to adjust the viscosity of the matrix for different means of application to a substrate. Viscosity can be regulated via the use of many different materials. One having skill in the art will be able to select viscosity or rheology modifiers to achieve different viscosity or rheology profiles for the matrix. According to one embodiment, the viscosity regulator may be a silicate compound or propylene glycol.

The matrix can be provided with an additional carrier. The additional carrier can be the same or different from any carrier used with the active ingredient component or any flavor carrier used with any flavor component. The additional carrier can further be selected to allow for the homogenization or formation of a solution or dispersion by the components of the matrix. Exemplary additional carriers include water, ethanol, propylene glycol, mixtures thereof, or the like.

The matrix can be provided with a sweetener to enhance the flavor of the active ingredient delivery system to the user. Any food-grade sweeteners are contemplated and may include, for example, sugar, cane, honey, agave, high-fructose corn syrup, allulose, maltitol, aspartame, syrup, saccharin, sorbitol, stevia, sucralose, and xylitol.

The various components of the matrix can be selected in different amounts and tuned by amount or component selection to control the rate of release of the active ingredient component from the active ingredient delivery system for desired effectiveness or intensity as a result of rate of delivery through a user's gums or cheek.

### ACTIVE INGREDIENT DELIVERY SYSTEM

In embodiments, the active ingredient delivery system comprises a matrix disposed onto a substrate. In preferred embodiments, the matrix includes an active ingredient component, a carrier component, and one or more of a flavor component, a softening agent, a viscosity regulator, an additional carrier, and a sweetener. In certain embodiments, the active ingredient delivery system has a moisture content of greater than 20 %, preferably greater than 25%, more preferably greater than 30 %, even more preferably greater than 35 %, still more preferably between 35 and 75 %, more preferably between 40 and 52 %, or between 44 and 50 %.

In some embodiments, a softening agent may be dosed onto the substrate separately or in addition to its incorporation via the matrix. In certain embodiments, the substrate is dosed with a softening agent prior to application of the matrix having an active ingredient component onto the substrate. A softening agent may be dosed onto the substrate in this way by dipping, printing, or any conventional method for dosing paper-based substrates with liquid.

In certain preferred embodiments, the matrix is print applied to the substrate less than ten times, in more preferred embodiments less than 7 times, in more preferred embodiments no more than five times, in more preferred embodiments, no more than four times, in more preferred embodiments, no more than three times. In certain preferred embodiments, the matrix is printed onto the substrate at least one time, in further embodiments, at least two times, and in still further embodiments at least three times. Given the contents of the present disclosure, a person having skill in the art will recognize that using a higher concentration of active ingredient component in a matrix may result in the need for fewer printing runs to achieve a certain total amount of active ingredient component in an active ingredient delivery system. One having skill in the art will recognize the benefits of fewer printing runs, coming in the form of decreased processing time and decreased processing cost. Fewer printing runs may, however, also yield a lower moisture content in the active ingredient delivery system, which can in turn result in a less desirable mouth-feel, among other effects. In embodiments for which a higher concentration of active ingredient component is used and fewer printing runs are used, the matrix may further include a softening agent to improve mouth-feel. One preferable such softening agent is glycerin, as discussed above. A person having skill in the art, with the benefit of this disclosure, will be able to balance active ingredient component concentration and any glycerin concentration to optimize the number of required printing runs and the appropriate mouth-feel for a user.

In certain embodiments, the matrix is disposed on the substrate to substantially cover the substrate in its entirety. In certain other embodiments, the matrix is applied to cover only part of the substrate.

Fig. 1 illustrates an exemplary active ingredient delivery system 10 comprising a matrix screen printed onto a first surface 25 of the substrate 12. The matrix may also be printed on the opposing second surface 26 of the substrate 12. In the exemplary active ingredient delivery system of Fig. 1, the active ingredient delivery system has perforations 24 and includes ten segments or strips, such as segments 20', 20", and 20''', separated by the perforations 24. The perforations 24 separating the segments 20 allow a user to easily tear off a portion providing an individual dose of an active ingredient component.

In other embodiments, the active ingredient delivery system is not perforated. For example the segments may be cut into individual dose portions. The active ingredient delivery system may have a variety of regular or irregular shapes as may fit in a user's mouth against their gums. Where such shapes have edges or corners, said edges or corners may be rounded, square, or have a different, suitable configuration.

Fig. 2 illustrates an embodiment of a segment or strip 20 of the active ingredient delivery system of the present invention. The segment may include a first side 31, a second opposing side 32, a first end 33, and a second opposing end 34. Additionally, the segment 20 may include a first surface 25 and an opposing second surface 26. As shown in Figs. 1 and 2, the segments are sized to fit on a user's gums and are dimensioned with a length 21, a width 23, and a depth 22.

The present disclosure also includes a method of making an active ingredient delivery system. The method comprises the steps of providing a paper-based substrate comprising fibers that are not dissolvable but are biodegradable, printing a matrix onto the paper-based substrate, the matrix comprising an active ingredient component and a carrier, and sizing the paper-based substrate having the matrix printed thereon to have a length, a width, and a depth dimension so that the paper-based substrate fits on a user's gums. The matrix further optionally includes one or more of a flavor component, a softening agent, a viscosity regulator, an additional carrier, and a sweetener.

In certain embodiments, the matrix provided by the method may have the characteristics of the active ingredient delivery system described in the disclosure.

In preferred embodiments of the method, the matrix is printed directly onto the substrate.

Suitable printing techniques include screen printing technology, flexo printing technology, inkjet/digital printing technology, tempo printing technology, gravure printing, relief printing, rotary printing, 3D printing, offset printing, spray preparation, or modified versions of these technologies.

In preferred embodiments, the step of printing a matrix onto the paper-based substrate is accomplished by screen printing.

Screen printing technique refers in this disclosure to a printing technique, wherein printing mixtures are rationed through a screen onto a product to be printed. Screen printing is also generally referred to as silkscreen printing, because the yarns of the mesh originally used in screen-printing were made of silk fiber. In principle, the disclosure contemplates a variety of screen-printing techniques, including the use of manual and semi-automatic techniques, as well as automated lines with screens and a printable product traveling thereon. The conditions of screen printing may vary as desired. For example, screen density can be adjusted to control the amount of matrix, and thus control the amount of active ingredient component and any flavor component to be printed on a substrate and included in an active ingredient delivery system. A denser screen will yield a lower rate of matrix transfer than will a coarse sieve and, correspondingly, the amount of active ingredient component on a printed substrate varies according to the screen density.

In certain embodiments, between each printing run, matrix or other liquid is reloaded onto the substrate to account for liquid lost to the screen. In preferred embodiments, liquid is re-loaded using electronics industry-grade dosing pumps at least once between printing runs.

Accordingly, the dosage of the active ingredient component in the active ingredient delivery system is precise, easy, and simple. For example, it is easy to ration a sufficient amount of an active ingredient component to a user by adjusting the amount of matrix delivered to a substrate for a given active ingredient delivery system. Further, since screen printing allows a more precise delivery of specific amounts of an active ingredient component via a matrix, with tighter tolerances than other methods like spraying or dipping, the active ingredient delivery system of the present disclosure can attain higher dosages that are permissible under the regulatory laws of a given jurisdiction for a given active ingredient.

Compared to the known art, the disclosed active ingredient delivery system has the special advantage of easily, accurately controlled distribution of an active ingredient component and any other matrix components (e.g., a flavor component) to a substrate.

The various components of the system of the present invention may be composed of any number of materials, and steps may be added to the disclosed methods as may be recognized by persons having skill in the art in light of this disclosure. Additional materials and variations in matrix application and printing methods may found in US Patent Pub. Nos. 2016/0137858 A1 and 2014/0349044 A1 and applied analogously in light of the present disclosure.

### EXAMPLE 1

In an example embodiment of the present invention, an absorbent substrate, having thickness 1.9 mm, and made up of cellulose pulp (50 - 60 wt. % of the substrate) and cotton linters (40 - 50 wt. % of the substrate) may be fed by sheet feeder (commonly used in the graphics industry) into a high-speed screen printing machine (Sakurai brand, Sakurai Graphic System Corp., Hounslow, Middlesex, UK). The mesh of the screen printing machine, preferably 063-063 lines, may be used to optimize delivery of the printing fluid (the matrix). A nicotine active ingredient component may be incorporated into a mixture that includes 10 wt. % nicotine (available from Nicobrand, 189 Castleroe Road, Coleraine, Co. Londonderry, BT51 3RP, UK) and the remainder propylene glycol carrier. The active ingredient component mixture may be pressed onto the substrate as many times as necessary to reach the desired nicotine content in the printed substrate (for example three runs). A flavor component of approximately 2 wt. % and a softening agent, such as food-grade glycerin, of approximately 30 wt. % (each available from Hertz Flavors GmbH & Co. KG) may be incorporated into a second mixture. The flavor component may be formed from food-grade, edible flavors and sweetener in solution. The remainder of the second mixture may include a propylene glycol carrier. The second mixture may be pressed onto the substrate as many times as necessary to achieve a desired moisture content of the product, taking into account the amount of active ingredient component mixture added (for example three runs). In this example, the desired moisture content may be 40 % by weight. After printing, the matrix-printed substrate may be cut using a die-cutting machine (available from Bobst Group SA, Mex, Switzerland) to a desired shape for a desired nicotine content in each dose. The cut, matrix-printed substrates may be further perforated to allow a user to easily tear off a piece of the matrix-printed substrate that includes an appropriate amount of nicotine for a single use.

In the example, all liquid ingredients are preferably mixed at room temperature. Standard, generally available equipment may be used for mixing. Mixing does not require mechanical mixing. The manufacturing process may be carried out at room temperature. The liquid raw materials used preferably do not evaporate at room temperature and thus do not require enhanced ventilation.

In the appended claims, reference to an element in the singular is not intended to mean "one and only one" unless explicitly so stated, but rather "one or more". Furthermore, no element, component, or combination in the present disclosure is intended to be dedicated to the public regardless of whether the element, component, or method step is explicitly recited in the claims. No claim element herein is to be construed under the provisions of 35 U.S.C. § 112, sixth paragraph, unless the element is expressly recited using the phrase "means for." Absent express definitions herein, all claim terms are to be given all ordinary and accustomed meanings that are not irreconcilable with the present specification and the file history.

Further, the purpose of the Abstract is to enable the various patent offices and the public generally, and especially the scientists, engineers, and practitioners in the art who are not familiar with patent or legal terms or phraseology, to determine quickly from a cursory inspection the nature and essence of the technical disclosure of the application. The Abstract is not intended to be limiting as to the scope of the invention in any way.

## Claims

1. An active ingredient delivery system comprising:
a paper-based substrate comprising fibers that are not dissolvable but are biodegradable; and
a matrix, wherein the matrix comprises an active ingredient component and a carrier,
wherein the matrix is disposed on the paper-based substrate by printing the matrix onto the substrate, and
wherein the paper-based substrate has a length, a width, and a depth dimension such that the paper-based substrate, having the matrix disposed thereon, fits on a user's gums.

2. The active ingredient delivery system of claim 1, wherein matrix further comprises a softening agent, optionally the softening agent is food-grade glycerin, and optionally the glycerin is present in an amount of 2 to 60 % by weight as a percentage of the matrix weight or in an amount of 15 to 45 % by weight as a percentage of the matrix weight.

3. The active ingredient delivery system of claim 1, wherein the paper-based substrate comprises cellulose pulp and cotton linters, optionally the paper-based substrate comprises at least about 40 % cellulose pulp by weight of the paper-based substrate and at least about 30% cotton linters by weight of the paper-based substrate, and/or at most about 70 % cellulose pulp by weight of the paper-based substrate and at most about 60% cotton linters by weight of the paper-based substrate, and/or the paper-based substrate comprises about 50 to about 60 % cellulose pulp by weight of the paper-based substrate, and/or about 40 to about 50 % cotton linters by weight of the paper-based substrate.

4. The active ingredient delivery system of claim 3, wherein the paper-based substrate is substantially free of viscose fibers.

5. The active ingredient delivery system of claim 1, wherein the paper-based substrate has a length of about 10 to about 50 mm, a width of about 3 to about 15 mm, and a depth of about 0.2 to about 2.0 mm.

6. The active ingredient delivery system of claim 1, wherein the active ingredient component is selected from nicotine, caffeine, THC, CBD, vitamins, ibuprofen, or a pharmaceutical, or mixtures thereof, and/or wherein the active ingredient component is selected from nicotine, THC, or CBD, vitamins, glucose, or mixtures thereof.

7. The active ingredient delivery system of claim 6, wherein the active ingredient component is nicotine.

8. The active ingredient delivery system of claim 7, wherein the active ingredient component comprises about 0.02 to about 30 wt. % of the matrix.

9. The active ingredient delivery system of claim 1, wherein the active ingredient component is dissolved or dispersed into the carrier before being disposed on the substrate, and/or wherein the carrier comprises propylene glycol.

10. The active ingredient delivery system of claim 1, wherein the matrix further comprises a flavor component, optionally the active ingredient delivery system further comprises a flavor carrier, wherein the flavor component is dissolved or dispersed into the flavor carrier before being disposed on the substrate, optionally the flavor carrier comprises propylene glycol.

11. The active ingredient delivery system of claim 10, wherein the matrix comprises about 0.2 to about 15 wt. % of the flavor component and about 0.02 to about 30 wt. % of the active ingredient component as a percentage of the total matrix weight.

12. The active ingredient delivery system of claim 1, wherein the matrix is disposed on the substrate via screen printing.

13. The active ingredient delivery system of claim 1, wherein the moisture content of the active ingredient delivery system is greater than about 20 %, and/or the moisture content of the active ingredient delivery system is 35-75 %.

14. The active ingredient delivery system of claim 1, further comprising multiple paper-based substrates each having the matrix disposed thereon, wherein the multiple paper-based substrates are connected to form a sheet and wherein a user can easily tear off a portion providing an individual dose of the active ingredient component, optionally the multiple paper-based substrates are connected via perforations.

15. The active ingredient delivery system of claim 1, wherein the paper-based substrate having the matrix disposed thereon is dimensioned such that it fits on a user's gums without forming a protrusion that is visible on the user's face.

16. The active ingredient delivery system of claim 1, wherein the matrix further comprises a sweetener, and/or the active ingredient delivery system further comprises a softening agent, a flavor component, and a sweetener, wherein the carrier, the softening agent, the flavor component, and the sweetener are food-grade.

17. An active ingredient delivery system comprising:
a paper-based substrate comprising multiple connected segments and fibers that are not dissolvable but are biodegradable; and
a matrix, wherein the matrix comprises an active ingredient component and a carrier;
wherein the matrix is printed on the paper-based substrate, and
wherein the multiple connected segments are connected to allow a user to easily separate a segment of the multiple connected segments of the paper-based substrate having matrix printed thereon, and wherein each segment of the multiple connected segments has a length, a width, and a depth dimension such that each segment fits on a user's gums, optionally the multiple connected segments are connected via perforations.

18. The active ingredient delivery system of claim 17 further comprising a flavor component and a softening agent, optionally the carrier, the softening agent, and the flavor component are food-grade.

19. The active ingredient delivery system of claim 17, wherein the matrix comprises a first liquid mixture and a second liquid mixture, wherein the first liquid mixture comprises the active ingredient component and the carrier and the second liquid mixture comprises the flavor component, the softening agent, and the carrier, wherein the first liquid mixture and the second liquid mixture are separately printed on the paper-based substrate.

20. A method of making an active ingredient delivery system, the method comprising the steps of:
providing a paper-based substrate comprising fibers that are not dissolvable but are biodegradable;
printing a matrix onto the paper-based substrate, the matrix comprising an active ingredient component and a carrier; and
sizing the paper-based substrate having the matrix printed thereon to have a length, a width, and a depth dimension so that the paper-based substrate fits on a user's gums.

21. The method of claim 20, wherein the active ingredient component is dissolved or dispersed in the carrier, and/or the matrix further comprises a softening agent, and/or the matrix further comprises a flavor component, optionally the matrix further comprises a flavor carrier and the flavor component is dissolved or dispersed into the flavor carrier, and/or the carrier comprises propylene glycol, and/or the step of printing the matrix onto the paper-based substrate is accomplished by screen printing, optionally the screen-printing further comprises reloading liquid onto the substrate between screen printing runs via at least two dosing pumps provided to distribute matrix onto the substrate.

22. A method of making an active ingredient delivery system, the method comprising the steps of:
providing a paper-based substrate comprising multiple connected segments and fibers that are not dissolvable but are biodegradable;
printing a matrix onto the paper-based substrate, the matrix comprising an active ingredient component and a carrier; and
sizing the multiple connected segments to allow a user to easily separate a segment of the multiple connected segments of the paper-based substrate having matrix printed thereon, wherein each segment of the multiple connected segments has a length, a width, and a depth dimension such that each segment fits on a user's gums, optionally the multiple connected segments are connected via perforations.

## Patentansprüche

1. Wirkstoffabgabesystem umfassend:
ein papierbasiertes Substrat, das Fasern umfasst, die nicht löslich sind, aber bioabbaubar sind; und
eine Matrix, wobei die Matrix eine Wirkstoffkomponente und einen Träger umfasst,
wobei die Matrix auf dem papierbasierte Substrat aufgebracht wird, indem die Matrix auf das Substrat aufgedruckt wird, und
wobei das papierbasierte Substrat eine Länge, eine Breite und ein Tiefenmaß aufweist, so dass das papierbasierte Substrat mit der darauf aufgebrachten Matrix auf das Zahnfleisch eines Benutzers passt.

2. Wirkstoffabgabesystem nach Anspruch 1, wobei die Matrix ferner ein Erweichungsmittel umfasst, wobei wahlweise das Erweichungsmittel ein lebensmitteltaugliches Glyzerin ist und wahlweise das Glyzerin in einer Menge von 2 bis 60 Gew.-% als prozentualer Anteil am Matrixgewicht oder in einer Menge von 15 bis 45 Gew.-% als prozentualer Anteil am Matrixgewicht vorliegt.

3. Wirkstoffabgabesystem nach Anspruch 1, wobei das papierbasierte Substrat Cellulosefaserstoff und Baumwolllinters umfasst, wobei wahlweise das papierbasierte Substrat mindestens etwa 40 % Cellulosefaserstoff bezogen auf das Gewicht des papierbasierten Substrats und mindestens etwa 30 % Baumwolllinters bezogen auf das Gewicht des papierbasierten Substrats, und/oder höchstens etwa 70 % Cellulosefaserstoff bezogen auf das Gewicht des papierbasierten Substrats und höchstens etwa 60 % Baumwolllinters bezogen auf das Gewicht das papierbasierten Substrats umfasst, und/oder das papierbasierte Substrat etwa 50 bis etwa 60 % Cellulosefaserstoff bezogen auf das Gewicht des papierbasierten Substrats und/oder etwa 40 bis etwa 50 % Baumwolllinters bezogen auf das Gewicht des papierbasierten Substrats umfasst.

4. Wirkstoffabgabesystem nach Anspruch 3, wobei das papierbasierte Substrat im Wesentlichen frei von Viskosefasern ist.

5. Wirkstoffabgabesystem nach Anspruch 1, wobei das papierbasierte Substrat eine Länge von etwa 10 bis etwa 50 mm, eine Breite von etwa 3 bis etwa 15 mm, und eine Tiefe von etwa 0,2 bis etwa 2,0 mm aufweist.

6. Wirkstoffabgabesystem nach Anspruch **Error! Reference source not found.,** wobei die Wirkstoffkomponente aus Nikotin, Kaffein, THC, CBD, Vitaminen, Ibuprofen oder einem Pharmazeutikum oder Mischungen derselben ausgewählt ist, und/oder wobei die Wirkstoffkomponente aus Nikotin, THC oder CBD, Vitaminen, Glukose oder Mischungen derselben ausgewählt ist.

7. Wirkstoffabgabesystem nach Anspruch 6, wobei es sich bei der Wirkstoffkomponente um Nikotin handelt.

8. Wirkstoffabgabesystem nach Anspruch 7, wobei die Wirkstoffkomponente etwa 0,02 bis etwa 30 Gew.% der Matrix umfasst.

9. Wirkstoffabgabesystem nach Anspruch 1, wobei die Wirkstoffkomponente im Träger gelöst oder dispergiert wird, bevor sie auf dem Substrat aufgebracht wird, und/oder wobei der Träger Propylenglykol umfasst.

10. Wirkstoffabgabesystem nach Anspruch 1, wobei die Matrix ferner eine Aromakomponente umfasst, wobei wahlweise das Wirkstoffabgabesystem ferner einen Aromaträger umfasst, wobei die Aromakomponente im Aromaträger gelöst oder dispergiert wird, bevor sie auf dem Substrat aufgebracht wird, wobei wahlweise der Aromaträger Propylenglykol umfasst.

11. Wirkstoffabgabesystem nach Anspruch 10, wobei die Matrix etwa 0,2 bis etwa 15 Gew.% der Aromakomponente und etwa 0,02 bis etwa 30 Gew.% der Wirkstoffkomponente als prozentualen Anteil am Matrixgesamtgewicht umfasst.

12. Wirkstoffabgabesystem nach Anspruch 1, wobei die Matrix per Siebdruck auf dem Substrat aufgebracht wird.

13. Wirkstoffabgabesystem nach Anspruch 1, wobei der Feuchtigkeitsgehalt des Wirkstoffabgabesystem größer als etwa 20 % beträgt und/oder der Feuchtigkeitsgehalt des Wirkstoffabgabesystem 35-75 % beträgt.

14. Wirkstoffabgabesystem nach Anspruch 1, das ferner mehrere papierbasierte Substrate umfasst, die jeweils die darauf aufgebrachte Matrix aufweisen, wobei die mehreren papierbasierten Substrate verbunden sind, um ein Blatt auszubilden, und wobei ein Benutzer leicht einen Teilabschnitt abreißen kann, der eine individuelle Dosis der Wirkstoffkomponente bereitstellt, wobei wahlweise die mehreren papierbasierten Substrate über Perforationen verbunden sind.

15. Wirkstoffabgabesystem nach Anspruch 1, wobei das papierbasierte Substrat mit der auf ihm aufgebrachten Matrix so bemessen ist, dass es auf das Zahnfleisch eines Benutzers passt, ohne eine im Gesicht des Benutzers sichtbare Ausbeulung auszubilden.

16. Wirkstoffabgabesystem nach Anspruch 1, wobei die Matrix ferner ein Süßungsmittel umfasst, und/oder das Wirkstoffabgabesystem ferner ein Erweichungsmittel, eine Aromakomponente und ein Süßungsmittel umfasst, wobei der Träger, das Erweichungsmittel, die Aromakomponente und das Süßungsmittel lebensmitteltauglich sind.

17. Wirkstoffabgabesystem umfassend:
ein papierbasiertes Substrat, das mehrere verbundene Segmente und Fasern, die nicht löslich sind, aber bioabbaubar sind, umfasst; und
eine Matrix, wobei die Matrix eine Wirkstoffkomponente und einen Träger umfasst;
wobei die Matrix auf dem papierbasierten Substrat aufgedruckt ist, und
wobei die mehreren verbundenen Segmente so verbunden sind, dass sie es einem Benutzer ermöglichen, ein Segment von den mehreren verbundenen Segmenten des papierbasierten Substrats mit dort aufgedruckter Matrix abzutrennen, und wobei jedes Segment der mehreren verbundenen Segmente eine Länge, eine Breite und ein Tiefenmaß aufweist, so dass jedes Segment auf das Zahnfleisch eines Benutzers passt, wobei wahlweise die mehreren verbundenen Segmente über Perforationen verbunden sind.

18. Wirkstoffabgabesystem nach Anspruch 17, ferner umfassend eine Aromakomponente und ein Erweichungsmittel, wobei wahlweise der Träger, das Erweichungsmittel und die Aromakomponente lebensmitteltauglich sind.

19. Wirkstoffabgabesystem nach Anspruch 17, wobei die Matrix ein erstes flüssiges Gemisch und ein zweites flüssiges Gemisch umfasst, wobei das erste flüssige Gemisch die Wirkstoffkomponente und den Träger umfasst und das zweite flüssige Gemisch die Aromakomponente, das Erweichungsmittel und den Träger umfasst, wobei das erste flüssige Gemisch und das zweite flüssige Gemisch separat auf dem papierbasierten Substrat aufgedruckt sind.

20. Verfahren zur Herstellung eines Wirkstoffabgabesystems, wobei das Verfahren die Schritte umfasst:
Bereitstellen eines papierbasierten Substrats, das Fasern umfasst, die nicht löslich sind, aber bioabbaubar sind;
Aufdrucken einer Matrix auf das papierbasierte Substrat, wobei die Matrix eine Wirkstoffkomponente und einen Träger umfasst; und
Bemessen des papierbasierten Substrats mit dort aufgedruckter Matrix dergestalt, dass es eine Länge, eine Breite und ein Tiefenmaß aufweist, so dass das papierbasierte Substrat auf das Zahnfleisch eines Benutzers passt.

21. Verfahren nach Anspruch 20, wobei die Wirkstoffkomponente im Träger gelöst oder dispergiert wird, und/oder die Matrix ferner ein Erweichungsmittel umfasst, und/oder die Matrix ferner eine Aromakomponente umfasst, wobei wahlweise die Matrix ferner einen Aromaträger umfasst und die Aromakomponente im Aromaträger aufgelöst oder dispergiert wird, und/oder der Träger Propylenglykol umfasst, und/oder der Schritt des Aufdruckens der Matrix auf das papierbasierte Substrat durch Siebdruck vollzogen wird, wobei wahlweise der Siebdruck ferner umfasst, dass die Flüssigkeit über mindestens zwei Dosierpumpen, die zur Verteilung der Matrix auf dem Substrat vorgesehen sind, zwischen Siebdruckgängen auf das Substrat nachchargiert wird.

22. Verfahren zur Herstellung eines Wirkstoffabgabesystems, wobei das Verfahren die Schritte umfasst:
Bereitstellen eines papierbasierten Substrats, das mehrere verbundene Segmente und Fasern, die nicht löslich sind, aber bioabbaubar sind, umfasst;
Aufdrucken einer Matrix auf das papierbasierte Substrat, wobei die Matrix eine Wirkstoffkomponente und einen Träger umfasst; und
Bemessen der mehreren verbundenen Segmente dergestalt, dass sie es einem Benutzer ermöglichen, ein Segment von den mehreren verbundenen Segmenten des papierbasierten Substrats mit dort aufgedruckter Matrix abzutrennen, wobei jedes Segment der mehreren verbundenen Segmente eine Länge, eine Breite und ein Tiefenmaß aufweist, so dass jedes Segment auf das Zahnfleisch eines Benutzers passt, wobei wahlweise die mehreren verbundenen Segmente über Perforationen verbunden sind.

## Revendications

1. Système de libération de substance active comprenant :
un substrat à base de papier comprenant des fibres qui ne sont pas solubles, mais qui sont biodégradables; et
une matrice, ladite matrice comprenant un composant de substance active et un vecteur,
dans lequel la matrice est disposée sur le substrat à base de papier par impression de la matrice sur le substrat, et
dans lequel le substrat à base de papier a une longueur, une largeur et une dimension de profondeur telles que le substrat à base de papier, avec la matrice disposée sur celui-ci, s'adapte à la gencive d'un utilisateur.

2. Système de libération de substance active selon la revendication 1, dans lequel la matrice comprend également un agent ramollissant, l'agent ramollissant étant optionnellement une glycérine de qualité alimentaire et la glycérine étant optionnellement présente dans un pourcentage de 2 à 60 % par rapport au poids de la matrice ou dans un pourcentage de 15 à 45 % par rapport au poids de la matrice.

3. Système de libération de substance active selon la revendication 1, dans lequel le substrat à base de papier comprend une pâte cellulosique et des linters de coton, le substrat à base de papier comprenant optionnellement au moins environ 40 % de pâte cellulosique par rapport au poids du substrat à base de papier et au moins environ 30 % de linters de coton par rapport au poids du substrat à base de papier, et/ou au maximum environ 70 % de pâte cellulosique par rapport au poids du substrat à base de papier et au maximum environ 60 % de linters de coton par rapport au poids du substrat à base de papier, et/ou le substrat à base de papier comprenant environ 50 à environ 60 % de pâte cellulosique par rapport au poids du substrat à base de papier et/ou environ 40 à environ 50 % de linters de coton par rapport au poids du substrat à base de papier.

4. Système de libération de substance active selon la revendication 3, dans lequel le substrat à base de papier est essentiellement exempt de fibres de viscose.

5. Système de libération de substance active selon la revendication 1, dans lequel le substrat à base de papier a une longueur d'environ 10 à environ 50 mm, une largeur d'environ 3 à environ 15 mm et une profondeur d'environ 0,2 à environ 2,0 mm.

6. Système de libération de substance active selon la revendication **Error! Reference source not found.,** dans lequel le composant de substance active est choisi parmi la nicotine, la caféine, le THC, le CBD, les vitamines, l'ibuprofène ou un produit pharmaceutique, ou des mélanges de ceux-ci, et/ou dans lequel le composant de substance active est choisi parmi la nicotine, le THC, le CBD, les vitamines, la glucose, ou des mélanges de ceux-ci.

7. Système de libération de substance active selon la revendication 6, dans lequel le composant de substance active est la nicotine.

8. Système de libération de substance active selon la revendication 7, dans lequel le composant de substance active comprend environ 0,02 à environ 30 % en poids de la matrice.

9. Système de libération de substance active selon la revendication 1, dans lequel le composant de substance active est dissout ou dispersé dans le vecteur avant d'être disposé sur le substrat, et/ou dans lequel le vecteur comprend du propylèneglycol.

10. Système de libération de substance active selon la revendication 1, dans lequel la matrice comprend également un composant d'arôme, le système de libération de substance active comprenant optionnellement également un vecteur d'arôme, dans lequel le composant d'arôme est dissout ou dispersé dans le vecteur d'arôme avant d'être disposé sur le substrat, le vecteur d'arôme comprenant optionnellement du propylèneglycol.

11. Système de libération de substance active selon la revendication 10, dans lequel la matrice comprend environ 0,2 à environ 15 % en poids du composant d'arôme et environ 0,02 à environ 30 % en poids du composant de substance active en pourcentage par rapport au poids total de la matrice.

12. Système de libération de substance active selon la revendication 1, dans lequel la matrice est disposée sur le substrat par sérigraphie.

13. Système de libération de substance active selon la revendication 1, dans lequel la teneur en humidité du système de libération de substance active est supérieure à environ 20 %, et/ou la teneur en humidité du système de libération de substance active est de 35-75 %.

14. Système de libération de substance active selon la revendication 1, comprenant également plusieurs substrats à base de papier comprenant chacun la matrice disposée sur ceux-ci, dans lequel les plusieurs substrats à base de papier sont reliés pour former une feuille et dans lequel un utilisateur peut facilement en arracher une portion fournissant une dose individuelle du composant de substance active, lesdits plusieurs substrats à base de papier étant optionnellement reliés via des perforations.

15. Système de libération de substance active selon la revendication 1, dans lequel le substrat à base de papier avec la matrice disposée sur celui-ci est dimensionné de telle sorte qu'il s'adapte à la gencive d'un utilisateur sans former de protubérance visible dans le visage de l'utilisateur.

16. Système de libération de substance active selon la revendication 1, dans lequel la matrice comprend également un édulcorant, et/ou le système de libération de substance active comprend également un agent ramollissant, un composant d'arôme et un édulcorant, dans lequel le vecteur, l'agent ramollissant, le composant d'arôme et l'édulcorant sont de qualité alimentaire.

17. Système de libération de substance active comprenant :
un substrat à base de papier comprenant plusieurs segments reliés et des fibres qui ne sont pas solubles, mais qui sont biodégradables; et
une matrice, ladite matrice comprenant un composant de substance active et un vecteur;
dans lequel la matrice est imprimée sur le substrat à base de papier, et
dans lequel les plusieurs segments reliés sont reliés de telle sorte qu'ils permettent à un utilisateur de séparer facilement un segment desdits plusieurs segments reliés du substrat à base de papier avec la matrice disposée sur celui-ci, et dans lequel chaque segment desdits plusieurs segments reliés a une longueur, une largeur et une dimension de profondeur telles que chaque segment s'adapte à la gencive d'un utilisateur, lesdits plusieurs segments reliés étant optionnellement reliés via des perforations.

18. Système de libération de substance active selon la revendication 17, comprenant également un composant d'arôme et un agent ramollissant, le vecteur, l'agent ramollissant et le composant d'arôme étant optionnellement de qualité alimentaire.

19. Système de libération de substance active selon la revendication 17, dans lequel la matrice comprend un premier mélange liquide et un second mélange liquide, dans lequel le premier mélange liquide comprend le composant de substance active et le vecteur et le second mélange liquide comprend le composant d'arôme, l'agent ramollissant et le vecteur, dans lequel le premier mélange liquide et le second mélange liquide sont imprimés séparément sur le substrat à base de papier.

20. Procédé de fabrication d'un système de libération de substance active, ledit procédé comprenant les étapes consistant à :
fournir un substrat à base de papier comprenant des fibres qui ne sont pas solubles, mais qui sont biodégradables;
imprimer une matrice sur le substrat à base de papier, ladite matrice comprenant un composant de substance active et un vecteur; et
dimensionner le substrat à base de papier avec la matrice imprimé sur celui-ci de telle sorte qu'il a une longueur, une largeur et une dimension de profondeur telles que le substrat à base de papier s'adapte à la gencive d'un utilisateur.

21. Procédé selon la revendication 20, dans lequel le composant de substance active est dissout ou dispersé dans le vecteur, et/ou la matrice comprend également un agent ramollissant, et/ou la matrice comprend également un composant d'arôme, la matrice comprenant optionnellement également un vecteur d'arôme et le composant d'arôme étant dissout ou dispersé dans le vecteur d'arôme, et/ou le vecteur comprend du propylèneglycol, et/ou l'étape d'impression de la matrice sur le substrat à base de papier est réalisée par sérigraphie, la sérigraphie comprenant optionnellement également le rechargement du liquide sur le substrat entre des passes sérigraphiques avec au moins deux pompes doseuses prévues pour distribuer la matrice sur le substrat.

22. Procédé de fabrication d'un système de libération de substance active, ledit procédé comprenant les étapes consistant à :
fournir un substrat à base de papier comprenant plusieurs segments reliés et des fibres qui ne sont pas solubles, mais qui sont biodégradables;
imprimer une matrice sur le substrat à base de papier, ladite matrice comprenant un composant de substance active et un vecteur; et
dimensionner les plusieurs segments reliés de telle sorte qu'ils permettent à un utilisateur de séparer un segment desdits plusieurs segments reliés du substrat à base de papier avec la matrice disposée sur celui-ci, dans lequel chaque segment desdits plusieurs segments reliés a une longueur, une largeur et une dimension de profondeur telles que chaque segment s'adapte à la gencive d'un utilisateur, lesdits plusieurs segments reliés étant optionnellement reliés via des perforations.
